# EUROPEAN PATENT SPECIFICATION

(11) **EP 0 555 346 B1**
(45) Date of publication and mention of the grant of the patent: **22.05.1996**
(21) Application number: 91920241.6
(22) Date of filing: 28.10.1991
(51) Int. Cl.: A61F 13/15

(54) **FIBROUS SUPERABSORBENT CORE HAVING INTEGRALLY ATTACHED HYDROPHOBIC FACING LAYER**
FASERFÖRMIGER, SUPERABSORBIERENDER KERN MIT INTEGRAL BEFESTIGTER, HYDROPHOBER ÜBERZUGSSCHICHT
NOYAU FIBREUX SUPERABSORBANT A COUCHE DE REVETEMENT HYDROPHOBE SOLIDAIRE

(30) Priority: 01.11.1990 US 608083
(43) Date of publication of application: 18.08.1993
(73) Proprietor: THE PROCTER & GAMBLE COMPANY, Cincinnati, Ohio 45202 (US)
(72) Inventor: AHR, Nicholas, Albert, Cincinnati, OH 45247 (US); OOTEN, David, Mark, Cincinnati, OH 45231 (US)
(74) Representative: Hirsch, Uwe Thomas
(86) International application number: PCT/US91/07988
(87) International publication number: WO 92/07534

(56) References cited:
- EP-A- 0 198 683
- EP-A- 0 336 578
- US-A- 5 015 245

## Description

### TECHNICAL FIELD

The present invention relates to absorbent structures comprising a fibrous superabsorbent core and an integrally attached hydrophobic facing layer.

Absorbent articles designed for absorbing body fluids typically comprise a hydrophillic absorbent core and a hydrophobic facing layer. The function of the facing layer is to wick body fluids away from the body into the absorbent core. Because the facing layer itself is hydrophobic, it will stay relatively dry, thus preventing the discomfort of a wet material in contact with the skin of the user of the absorbent article.

It is desirable to provide a good contact between the hydrophobic facing layer and the hydrophillic absorbent core, so as to enhance fluid transport from the facing layer to the core. It is further desirable to provide an absorbent core having a low bulk, yet a high absorbent capacity. It is further desirable that the absorbent core exhibits good wicking of fluid in the x- and y-directions.

It is therefore an object of the present Invention to provide a fibrous superabsorbent core with an integrally attached hydrophobic facing layer. It is a further object of this invention to provide such a core having low bulk and yet a high absorbent capacity.

### BACKGROUND ART

U.S. Patent 3,067,747, issued December 11, 1962 to Wolterding et al. discloses absorbent nonadherent bandages for surgical or menstrual use. The bandages have topsheets of nonwoven web of bonded synthetic hydrophobic fibers. The absorbent web may be a combination of cotton and/or rayon fibers mixed with thermoplastic fibers. The topsheet is thermally bonded to the absorbent web.

U.S. Patent 4,047,534, issued September 13, 1977 to Thomaschefsky et al. relates to nursing pads having an inner absorbent layer including a proportion of synthetic thermoplastic polymer fibers and an outer layer of thermoplastic polymer fibers. The layers are combined by embossing with heat and low pressure.

U.S. Patent 4,397,644, issued August 9, 1983 to Matthews et al. discloses a sanitary napkin disclosing a topsheet having a hydrophilicity gradient and an absorbent core. The topsheet consists of two layers. The top layer is thermoplastic, for example, spun bonded polypropylene. The so called transfer layer contains from 40 to 100 percent thermoplastic fibers. The top layer or cover is integrally attached to the transfer layer by heat, such as by hot calender embossing.

EP-A-336 578 discloses an absorbent product having an absorbent core comprising optionally fibrous superabsorbent material overlayed by a tissue, a wipe acquisition sheet and a topsheet.

U.S. Patent 4,844,965, issued July 4, 1989 to Foxman, relates to an absorptive device for incontinent patients. The device includes a liquid permeable absorptive member having an outer facing layer of synthetic fabric and an inner backing layer of fabric having a blend of thermal plastic and cellulose fibers. The synthetic outer facing layer is ultrasonically welded to the thermal plastic fibers of the blended material inner backing layer.

U.S. Patent 4,844,965, issued July 4, 1989 to Foxman, discloses an absorptive member (for example a bed pad) having an outer facing layer of thermal plastic material and an inner facing layer which has a material blend of thermal plastic and cellulose fibers. This synthetic outer facing layer is ultrasonically welded to the thermal plastic fibers of the blended material inner backing layer. The pad is washable.

U.S. Patent 4,886,697, issued December 12, 1989 to Perdelwitz, Jr. et al. relates to materials having at least one layer comprising a mixture of thermoplastic and other fibers which may be covered with thermoplastic material containing cover sheets. The materials are densified in discrete areas, and eventually cut within the densified region. The layer comprising a mixture of thermoplastic and other fibers may additionally contain a superabsorbent material. The facing layer may be thermobonded to the absorbent layer by pulling heated air through the web plus facing layer. The material is disclosed to be suitable for use in children's car seats.

U.S. Patent 4,939,017, issued July 3, 1990 to Foxman discloses an absorbent pad having an outer layer of 100 percent synthetic thermal plastic fabric and an absorptive layer which is a blend of plastic and cellulose fibers. The layers are ultrasonically welded.

### DETAILED DESCRIPTION OF THE INVENTION

The absorbent structures of the present invention comprise a fibrous superabsorbent core with an integrally attached hydrophobic facing layer. Specifically, the absorbent core comprises from about 5 percent to about 95 percent of superabsorbent fibers, and from about 5 percent to about 95 percent of a synthetic thermoplastic fibers. The absorben structur has a thickness from 0.3 mm to 2 mm. The hydrophobic facing layer consists essentially of synthetic hydrophobic thermoplastic fibers. The structure may be formed on a conventional carding machine, using one card for the hydrophobic facing layer, and one or more cards (up to about 20) for the absorbent core. The layers are integrally bonded by a thermal process. Preferably the bonding is carried out with heated calender rolls or using ultrasonic sealing. This has the additional advantage that during the bonding the bulk of the absorbent structure may be significantly reduced. For example, the caliper of the absorbent structures may be reduced by a factor of 5 to 15 during the thermobonding process. Preferably, the bonding pattern is discontinuous, as is the case when heated embossing rolls are used. In areas where the fibers are not bonded they remain soft and flexible. Moreover, in unbonded areas the superabsorbent fibers absorb fluid and swell more freely than in bonded areas.

The synthetic hydrophobic fibers of the facing layer may be any of such fibers known in the art for this type of application. Polyolefin fibers are preferred, with polypropylene fibers being highly preferred.

The thermoplastic fiber component of the absorbent core may be a hydrophilic or a hydrophobic fiber. If a hydrophobic fiber is used it may be the same or different than the hydrophobic fiber of the facing layer.

Any superabsorbent fiber known in the art may be used as a superabsorbent fiber in the absorbent core. Superabsorbent fibers may be formed by forming a water soluble superabsorbent polymer into water soluble filaments, contacting the filaments with a primary air stream having a velocity effective to attenuate and to partially dry the filaments, and contacting the attenuated filaments with a secondary air stream having a velocity effective to fragment the filaments into fibers. Particularly suitable superabsorbent polymers are polymers comprising a blend of (1) a copolymer of at least one alpha, beta-unsaturated carboxylic monomer and at least one monomer copolymerizable therewith, and (2) a cross-linking agent having crosslinking functionality comprising hydroxyl or heterocyclic carbonate groups. Highly preferred are maleic anhydride/isobutylene copolymers crosslinked with propylene carbonate or a mixture of pentaerythriol and butanediol.

Particularly preferred for use herein is Fibersorb, a commercially available superabsorbent fiber from Arco Chemical Company of Newton Square, Pennsylvania. These fibers are disclosed more fully in U.S. Patent No. 4,855,179, issued August 8, 1989, to Bourland et al., the disclosures of which are incorporated herein by reference. In addition to the hydrophobic fibers and the superabsorbent fibers, the absorbent core may further comprise additional absorbent fibers, like pulp fibers, cotton fibers or rayon. Preferred absorbent cores comprise from about 30 to about 70 percent hydrophobic fibers and from about 70 percent to about 30 percent superabsorbent fibers.

It is advantageous to use calender rolls having an embossing pattern for the thermobonding of the two layers. An example of a suitable embossing pattern is one having a wafer-like or diamond shape pattern of the type disclosed in U.S. Patent No. 4,781,710, issued November 1, 1988 to Megisan et al., the disclosures of which are incorporated herein by reference.

The absorbent structures of the present invention can be made very thin, i.e., having a thickness of less than about 2 millimeters, preferably less than about 1 millimeter, typically from about 0.3 to about 2 millimeters. Yet, the absorbent structures may have an absorbent capacity of more than 0.05 grams of a 0.9 percent saline solution per square centimeter of the absorbent structure. Typically, the absorbent capacity is in the range of from about 0.07 grams per square centimeter to about 0.25 grams per square centimeter for a structure having a thickness of 1 millimeter.

Preferred absorbent structures have a density in the range from about 0.08 grams per cubic centimeter to about 0.25 grams per cubic centimeter.

The absorbent structures of the present invention are particularly suitable for use in so called pantiliners. Pantiliners are absorbent pads used for the absorption of menstrual fluid during those days of the menstrual cycle that the discharge of menstrual fluid is low. Pantiliners are also commonly used for the absorption of vaginal discharge other than menses.

A pantiliner of the state of the art typically comprises a hydrophobic liquid pervious topsheet, a thin absorbent core, typically comprised of wood pulp fibers, and a liquid impervious backsheet, commonly a polyethylene film. The outer surface of the liquid impermeable backsheet may be provided with strips or blots of a pressure sensitive adhesive for fastening pantiliner in the crotch of the panties of the wearer. Commonly this pressure sensitive adhesive is protected from contamination and inadvertent adherence with a release liner. The release liner is to be removed by the user prior to adhering pantiliner to the panty. Pantiliners may have a length in the range of from 12 centimeters to about 16 centimeters, and a width of from about 3 centimeters to about 8 centimeters. A particularly desirable pantiliner has a slight dog bone shape, a length of about 14 centimeters, a narrowest width in its center portion of about 4 centimeters, and a greatest width near both ends of about 7 centimeters.

Examples of prior art pantiliners are disclosed in U.S. Patent No. 4,738,676, issued April 19, 1988 to Osborn III, the disclosures of which are incorporated herein by reference.

The absorbent structures of the present invention replace the hydrophobic topsheet and the absorbent core of a conventional pantiliner. It is desirable to provide the absorbent structure with a liquid impervious backsheet. This backsheet may be a suitable film of polyethylene or polypropylene. The backsheet can be thermally bonded or adhesively bonded to the absorbent structure. For adhesive bonding, a preferred adhesive pattern is a spiral spray or meltblown ("angel hair") pattern.

It is desirable to seal the absorbent structure around its perimeter, to prevent superabsorbent fibers from coming into contact with the skin of the wearer. Thermal embossing is a suitable method for sealing the perimeter of absorbent structures of the present invention.

### DETAILED DESCRIPTION OF A PREFERRED EMBODIMENT

The following three nonwoven webs are prepared on a nonwoven carding line. A polypropylene web having a basis weight of about 1 ounce per square yard (about 30 grams per square meter) and two nonwoven webs of a 50/50 blend of polypropylene fibers and Fibersorb superabsorbent fiber, each having a basis weight of about 1 ounce per square yard (about 30 grams per square meter). The three webs are combined, such that the all polypropylene layer is the top layer. The combined webs have a thickness of about 1/2 inch (about 12 millimeters). The combined webs are then calendered at a web speed of about 25 to 30 feet per minute (about 7.5 to 9 meters per minute) and at a temperature of from 140°C to about 165°C, preferably from about 150°C to about 160°C. The temperature should be increased when working at higher web speeds, and decreased when working at lower web speeds, to maintain good results. The calender pressure is about 160 pounds per linear inch (about 29 kilogram per linear centimeter). The calender rolls have an embossing pattern of a diamond shape, of the type described in U.S. Patent No. 4,781,710. The resulting absorbent structure has a caliper of about 0.8 millimeters. It has an absorbent capacity for a 0.9 percent saline solution of about 0.12 grams per square centimeter. Properly dimensioned strips of the absorbent structure are combined with a polyethylene backsheet material and provided with a panty fastening adhesive in the manner described hereinabove for conventional pantiliners. The resulting pantiliner is thin and flexible, yet has adequate absorbent capacity.

In an alternate embodiment the liquid impermeable backsheet is formed as follows. Prior to calendering the nonwoven webs described hereinabove are combined with an additional web consisting essentially of heat fusible fibers, for example, polyethylene fibers. This bottom layer is subsequently rendered liquid impermeable by heat fusing the fibers, for example, using a heated calender roll or Infrared radiation.

Similar structures are formed using 1 layer of polypropylene fibers and 3 layers of an 85/15 mixture of polypropylene/Fibersorb fibers.

## Claims

1. An absorbent structure comprising
a core, said core comprising fibrous superabsorbent materials; and
a hydrophobic facing layer;
said absorbent structure being characterised in that said facing layer is integrally attached to said core by a thermal process; and
said absorbent structure having a thickness of 0.3 mm to 2 mm.

2. An absorbent structure according to Claim 1 wherein the hydrophobic facing layer consists essentially of synthetic hydrophobic thermoplastic fibers, and the absorbent core comprises from about 5% to about 95% of superabsorbent fibers and from about 5% to about 95% of synthetic thermoplastic fibers.

3. An absorbent structure according to Claim 2, having an absorbent capacity for a 0.9% saline solution of from about 0.05 grams to about 0.25 grams per square centimeter of absorbent structure.

4. An absorbent structure according to Claim 2, wherein the synthetic hydrophobic thermoplastic fiber is a polyolefin fiber.

5. An absorbent structure according to Claim 2, wherein the superabsorbent fibers comprise a copolymer of maleic anhydride and isobutylene.

6. An absorbent structure according to Claim 2 wherein the absorbent core comprises from about 30% to about 70% of superabsorbent fiber and from about 30% to about 70% synthetic thermoplastic fiber.

7. An absorbent structure according to Claim 6, wherein the superabsorbent fiber comprises a copolymer of maleic anhydride and isobutylene.

8. An absorbent structure according to Claim 6, wherein the synthetic hydrophobic thermoplastic fiber is a polypropylene fiber.

9. A pantiliner comprising the absorbent structure of Claim 1, and a liquid impervious backsheet.

10. A pantiliner comprising the absorbent structure of Claim 6, and a liquid impervious backsheet.

11. A process for making an absorbent structure according to claims 1 to 10, comprising a fibrous superabsorbent core and an integrally attached hydrophobic facing layer, said process comprising the steps of:
a) forming a nonwoven web consisting essentially of synthetic hydrophobic thermoplastic fibers;
b) forming from one to 20 nonwoven webs comprising from about 5% to about 95% of a superabsorbent fiber and from about 5% to about 95% of a synthetic thermoplastic fiber;
c) combining the layers formed in steps a) and b) into a stack;
d) heat calendering the stack of step c) to a thickness of from about 0.3 millimeters to about 2 millimeters.

12. The process of Claim 11 wherein an embossing pattern is imparted to the absorbent structure during the heat calendering step d).

13. The process of Claim 11 wherein the superabsorbent fiber comprises a copolymer of isobutylene and maleic anhydride, and the thermoplastic hydrophobic fiber is polypropylene.

## Patentansprüche

1. Eine absorbierende Struktur, umfassend:
. einen Kern, wobei der genannte Kern faserige superabsorbierende Materialien umfaßt; und
. eine hydrophobe Umhüllungsschichte;
wobei die genannte absorbierende Struktur dadurch gekennzeichnet ist, daß die genannte Umhüllungsschichte durch ein thermisches verfahren an den genannten Kern integriert gebunden ist; und die genannte absorbierende Struktur eine Dicke von 0,3 mm bis 2 mm aufweist.

2. Eine absorbierende Struktur nach Anspruch 1, worin die hydrophobe Umhüllungsschichte im wesentlichen aus synthetischen hydrophoben thermoplastischen Fasern besteht und der absorbierende Kern zu etwa 5 % bis etwa 95 % superabsorbierende Fasern und zu etwa 5 % bis etwa 95 % synthetische thermoplastische Fasern umfaßt.

3. Eine absorbierende Struktur nach Anspruch 2 mit einer Absorptionskapazität für eine 0,9%-ige Salzlösung von etwa 0,05 Gramm bis etwa 0,25 Gramm pro Quadratzentimeter absorbierender Struktur.

4. Eine absorbierende Struktur nach Anspruch 2, worin die synthetische hydrophobe thermoplastische Faser eine Polyolefin-Faser ist.

5. Eine absorbierende Struktur nach Anspruch 2, worin die superabsorbierenden Fasern ein Copolymer von Maleinsäureanhydrid und Isobutylen umfassen.

6. Eine absorbierende Struktur nach Anspruch 2, worin der absorbierende Kern etwa 30 % bis etwa 70 % superabsorbierende Faser und etwa 30 % bis etwa 70 % synthetische thermoplastische Faser umfaßt.

7. Eine absorbierende Struktur nach Anspruch 6, worin die superabsorbierende Faser ein Copolymer von Maleinsäureanhydrid und Isobutylen umfaßt.

8. Eine absorbierende Struktur nach Anspruch 6, worin die synthetische hydrophobe thermoplastische Faser eine Polypropylen-Faser ist.

9. Eine Slipeinlage, die die absorbierende Struktur nach Anspruch 1 und ein flüssigkeitsundurchlässiges Rückenblatt umfaßt.

10. Eine Slipeinlage, die die absorbierende Struktur nach Anspruch 6 und ein flüssigkeitsundurchlässiges Rückenblatt umfaßt.

11. Ein Verfahren zur Herstellung einer absorbierenden Struktur nach den Ansprüchen 1 bis 10, die einen faserigen superabsorbierenden Kern und eine an den Kern integriert gebundene hydrophobe Umhüllungsschichte umfaßt, welches Verfahren folgende Schritte umfaßt:
a) Bilden einer nichtgewebten Bahn, die im wesentlichen aus synthetischen hydrophoben thermoplastischen Fasern besteht;
b) Bilden von ein bis 20 nichtgewebten Bahnen, die zu etwa 5 % bis etwa 95 % eine superabsorbierende Faser und zu etwa 5 % bis etwa 95 % eine synthetische thermoplastische Faser umfassen;
c) Vereinigen der in den Schritten a) und b) gebildeten Schichten zu einem Stapel;
d) Hitzekalandrierung des Stapels von Schritt c) auf eine Dicke von etwa 0,3 Millimeter bis etwa 2 Millimeter.

12. Das Verfahren nach Anspruch 11, in welchem während des Hitzekalandrierschrittes d) die absorbierende Struktur mit einem Prägemuster versehen wird.

13. Das Verfahren nach Anspruch 11, in welchem die superabsorbierende Faser ein Copolymer von Isobutylen und Maleinsäureanhydrid umfaßt und die thermoplastische hydrophobe Faser Polypropylen ist.

## Revendications

1. Structure absorbante comprenant :
une âme, ladite âme comprenant des matériaux fibreux superabsorbants; et
une couche de surface hydrophobe ;
ladite structure absorbante étant caractérisée en ce que ladite couche de surface est reliée intégralement à ladite âme à l'aide d'un procédé thermique; et
ladite structure absorbante ayant une épaisseur allant de 0,3 mm à 2mm.

2. Structure absorbante selon la revendication 1, dans laquelle la couche de surface hydrophobe est essentiellement constituée de libres synthétiques, hydrophobes, thermoplastiques et l'âme absorbante comprend d'environ 5% à environ 95% de libres superabsorbantes et d'environ 5% à environ 95% de fibres synthétiques thermoplastiques.

3. Structure absorbante selon la revendication 2 , ayant une capacité absorbante pour une solution saline à 0,9% allant de 0,05 gramme environ à 0,25 gramme environ par centimètre carré de structure absorbante.

4. Structure absorbante selon la revendication 2, dans laquelle les fibres synthétiques, hydrophobes thermoplastiques, sont des fibres polyoléfiniques.

5. Structure absorbante selon la revendication 2, dans laquelle les fibres superabsorbantes sont constituées d'un copolymère d'anhydride maléique et d'isobutylène.

6. Structure absorbante selon la revendication 2, dans laquelle l'âme absorbante est constituée d'environ 30% à environ 70% de fibres superabsorbantes et d'environ 30% à environ 70% de fibres synthétiques thermoplastiques.

7. Structure absorbante selon la revendication 6, dans laquelle les fibres superabsorbantes sont constituées d'un copolymère d'anhydride maléique et d'isobutylène.

8. Structure absorbante selon la revendication 6, dans laquelle les libres synthétiques, hydrophobes, thermoplastiques sont des fibres en polypropylène.

9. Garniture de slip comportant la structure absorbante selon la revendication 1 et une feuille de fond imperméable aux liquides.

10. Garniture de slip comportant la structure absorbante selon la revendication 6 et une feuille de fond imperméable aux liquides.

11. Procédé pour fabriquer une structure absorbante selon les revendications 1 à 10 comprenant une âme fibreuse superabsorbante et une couche de surface hydrophobe reliée intégralement, ledit procédé comprenant les étapes suivantes;
a) on forme une bande non tissée essentiellement constituée de fibres synthétiques, hydrophobes, thermoplastiques;
b) on forme d'une à 20 bandes non tissées comprenant d'environ 5% à environ 95% d'une fibre superabsorbante et d'environ 5% à environ 95% d'une libre synthétique thermoplastique;
c) on combine les couches formées dans les étapes a) et b) pour former une pile;
d) on effectue un calandrage à chaud de la pile de l'étape c) jusqu'à une épaisseur allant d'environ 0,3 millimètre à environ 2 millimètres.

12. Procédé selon la revendication 11 dans lequel un motif gaufré est communiqué à la structure absorbante au cours de l'étape de calandrage à chaud d).

13. Procédé selon la revendication 11 dans lequel la fibre superabsorbante est constituée d'un copolymère d'isobutylène et d'anhydride maléique et la libre thermoplastique, hydrophobe est constituée par du polypropylène.
